# EUROPEAN PATENT APPLICATION

(11) **EP 4 548 769 A1**
(43) Date of publication of application: **07.05.2025**
(21) Application number: 24210640.9
(22) Date of filing: 04.11.2024
(51) Int. Cl.: A23C 9/12, A23C 9/18, C12N 9/38, A23C 9/16

(54) **METHOD, DEVICE, AND SYSTEM FOR MANUFACTURING MILK POWDER, AND MILK POWDER OBTAINABLE BY SAID METHOD**

(30) Priority: 03.11.2023 NL 2036180
(71) Applicant: Vreugdenhil Groep B.V., 3861 PS Nijkerk (NL)
(72) Inventor: van der Zee, Douwe, 3861PS Nijkerk (NL)
(74) Representative: Arnold & Siedsma

(57) **Abstract**

The present invention relates to a method for manufacturing milk powder comprising at most 5 dry wt.% lactose, a device for manufacturing milk powder comprising at most 5 dry wt.% lactose, a system for manufacturing milk powder comprising at most 5 dry wt.% lactose, and milk powder obtainable by the method according to the invention.

## Description

The present invention relates to a method for manufacturing milk powder comprising at most 5 dry wt.% lactose, a device for manufacturing milk powder comprising at most 5 dry wt.% lactose, a system for manufacturing milk powder comprising at most 5 dry wt.% lactose, and milk powder obtainable by the method according to the invention.

Over the last decades there has been an increasing interest in sugar reductions in food products, to achieve healthier food products. In addition, there is an increasing interest in food products suitable for consumers with dietary requirements. For example, consumers which suffer from gluten intolerance and/or lactose intolerance.

Indeed, food products having a low lactose content or which are lactose free have been available in the market for many years.

Conventional milk products comprising a low lactose content and/or no lactose content are achieved using membrane filtration and/or enzymes for the hydrolysis of the lactose. A problem of the conventional methods and/or devices is that the taste and/or mouthfeel of the milk product decreases. This is in particular the case for milk products which include lactose free milk powder and/or milk powder with a low lactose content.

These problems prevent an efficient and effective manufacturing of lactose free milk products and/or milk products with a low lactose content. In addition, the problems prevent a pleasant consumption of lactose free milk products and/or milk products with a low lactose content by consumers.

An objective of the present invention is to provide a method for manufacturing milk powder that obviates or at least reduces the aforementioned problems and/or is more effective compared to conventional methods, devices, and systems.

This objective is achieved with the method for manufacturing milk powder comprising at most 5 dry wt.% lactose, comprising the steps of:
- providing a milk substrate to a reactor, wherein the milk in the reactor is heated to a temperature in the range of 30 °C to 80 °C; and
- adding a lactase to the reactor in an amount in the range of 0.1 wt.% of 3 wt.%,
wherein the residence time of the milk substrate and the lactase in the reactor is in the range of 20 minutes to 180 minutes.

It is noted that milk powder comprising at most 5 dry wt.% lactose refers to milk powder comprising 0 dry wt.% lactose to at most 5 dry wt.% lactose.

The method according to the invention for manufacturing milk powder comprising at most 5 dry wt.% lactose may start with the step of providing a milk substrate to a reactor, wherein the milk in the reactor may be heated to a temperature in the range of 30 °C to 80 °C. Said step of providing a milk substrate to a reactor may be followed by the step of adding a lactase to the reactor in an amount in the range of 0.1 wt.% of 3 wt.%. The step of adding a lactase is preferably performed during the step of heating in the reactor.

It is noted that the amount of lactase added to the reactor is defined as a dry weight percentage, wherein the dry weight of the lactase is defined as the weight percentage of the lactose amount in the milk substrate. In other words, the lactose amount in the milk substrate is used to determine the amount of lactase added to the reactor.

The method according to the invention enables to reduce the lactose content in a milk substrate. As a result, a (final) milk substrate, such as milk powder, comprising at most 5 dry wt.% lactose is achieved in an efficient and effective manner.

An advantage of the method according to the invention is that a (final) milk substrate, such as milk powder, suitable for consumption by lactose intolerant consumers is achieved. Furthermore, the method according to the invention enables to produce a substantially lactose free milk substrate for an affordable price. Therefore, consumers with a lactose intolerance may include (more of) said milk substrate in their diet, and thus a healthy diet is more affordable.

In a presently preferred embodiment according to the invention, the heating of the milk in the reactor may be performed at a temperature in the range of 40 °C to 70 °C, preferably at a temperature in the range of 45 °C to 60 °C, more preferably at a temperature in the range of 50 °C to 55 °C.

It was found that heating the milk in the reactor to a temperature in the range of 40 °C to 70 °C, preferably at a temperature in the range of 45 °C to 60 °C, more preferably at a temperature in the range of 50 °C to 55 °C provides an efficient and effective environment for catalysing the breakdown of lactose by the lactase.

In a further presently preferred embodiment according to the invention, the lactase may be added in an amount in the range of 0.1 wt.% to 2 wt.%, preferably in an amount in the range of 0.2 wt.% to 1.5 wt.%, more preferably in an amount in the range of 0.3 wt.% to 1 wt.%, even more preferably in an amount in the range of 0.3 wt.% to 0.6 wt.%.

It is noted that the weight percentage of lactase is based on the lactose content present in the milk substrate.

An advantage of the method according to the invention is that a relatively small amount of lactase (for example an amount in the range of 0.3 wt.% to 0.6 wt.%) may be used to manufacture milk powder comprising at most 5 dry wt.% lactose.

Another advantage of said lactase amount is that the

In a further presently preferred embodiment according to the invention, the residence time of the milk substrate and the lactase in the reactor may be in the range of 30 minutes to 150 minutes, preferably in the range of 40 minutes to 120 minutes, more preferably in the range of 50 minutes to 90 minutes, most preferably in the range of 60 minutes to 90 minutes.

It was found that the method according to the invention significantly reduces the time to manufacture milk powder comprising at most 5 dry wt.% lactose compared to conventional methods. In particular, the residence time of the milk substrate and the lactase in the reactor enables an efficient and effective method for manufacturing milk powder comprising at most 5 dry wt.% lactose.

In a further presently preferred embodiment according to the invention, the heating of the milk substrate in the reactor may be performed at a temperature in the range of 50 °C to 55 °C, the adding of lactase may comprise adding lactase in an amount in the range of 0.3 wt.% to 0.6 wt.%, and the residence time of the milk substrate and the lactase in the reactor may be in the range of 60 minutes to 90 minutes.

An advantage of the combination of heating the milk substrate in the reactor to a temperature in the range of 50 °C to 55 °C, adding lactase in an amount in the range of 0.3 wt.% to 0.6 wt.%, and having the residence time of the milk substrate and the lactase in the reactor in the range of 60 minutes to 90 minutes is that milk powder with the desired taste is achieved efficiently and effectively.

Yet another advantage of the method according to the invention is that the proteins, fat, and/or other components in the milk remain substantially intact/are substantially unchanged. Therefore, the desired taste and/or texture of the milk powder is preserved compared to conventional milk powder (milk powder including the starting amount of lactose). In addition, the milk powder obtained by the method according to the invention may be used in products suitable for human consumption. Said milk powder may reduce or obviate the use flavour enhancers as the taste and/or texture of the milk powder according to the invention substantially matches or closely matches the taste and/or texture of conventional milk powder (i.e. milk powder comprising lactose).

In a further presently preferred embodiment according to the invention, the lactase is a bèta-galactosidase. Preferably, the lactase enzyme (P-galactosidase) used was a Neutral Bacterial Enzyme indirectly obtained from Lactobacillus bulgaricus, and this enzyme is active in a temperature range of approximately 50 °C to 70 °C. The enzyme had an activity of 15250 SD lactase units g⁻¹ or more.

In a further presently preferred embodiment according to the invention, the lactase may be one or more selected from the group of GOGO YNL-1, preferably the lactase is GODO YNL-2.

It was found that the lactase used in the method according to the invention enables a (fast) hydrolysis.

Preferably Bonlacta^{™} as lactase is used in the method according to the invention.

The lactase, preferably GODO YNL-1 and/or GODO YNL-2, allows for process optimization and cost reduction. As a result, a wide range of lactose-free dairy products, such as lactose-free milk powder, can be manufactured at competitive costs.

Furthermore, the lactase (such as Bonlacta^{™}) used in the method according to the invention is fast acting under refrigeration conditions and is able to withstand high temperatures to enable fast lactose hydrolysis of a milk substrate. Said lactase comprises unique thermal properties which enables flash pasteurization or high-temperature short-time (HTST) processing.

In addition, the lactase used in the method according to the invention comprises a high purity. As a result, said lactase ensures a clean taste in products stored at refrigerated or ambient temperatures. Less undesired off-flavours and less undesired reactions in the milk substrate and/or milk powder bring opportunities for use in a wide variety of lactose-free (new) products.

The lactase according to the invention is preferably enabled to withstand temperatures up to 80 °C.

In a preferred embodiment, the method according to the invention comprises the step of adding salt to the milk substrate before the milk substrate in the reactor is heated. Preferably, the salt may be one or more selected from the group of sodium chloride, potassium chloride, sodium iodide, potassium iodide.

It was found that the lactose hydrolysis process may be further accelerated by providing salt to the milk substrate. In particular, the hydrolysis of the lactose in the milk substrate is increased when Bonlacta^{™} as lactase is used.

Furthermore, due to the activity of the lactase used in the method according to the invention, a relatively small dose needs be added to the milk substrate compared to conventional methods for reducing the lactose content in a milk substrate.

In a further presently preferred embodiment according to the invention, the method according to the invention further comprises the step of controlling the temperature in the reactor, and/or controlling the addition of the lactase, and/or controlling residence time in the reactor.

The step of controlling the temperature in the reactor, and/or controlling the addition of the lactase, and/or controlling the residence time in the reactor may result in adjusting the method according to the invention such that the desired lactose hydrolysis conditions are achieved.

An advantage of said steps is that an operator may, therewith, adjust the method according to the invention. Furthermore, said controlling may provide the desired milk powder in an efficient and effective manner.

In a further presently preferred embodiment according to the invention, the method according to the invention further comprises a first pasteurisation of the milk substrate and/or a second pasteurisation of the milk substrate, wherein the first pasteurisation may be performed before the step of providing a milk substrate to a reactor, and the second pasteurisation may be performed after the step of adding a lactase to the reactor. Preferably, the first pasteurisation and the second pasteurisation may be independently from each other be performed at a temperature in the range of 70 °C to 100 °C, preferably a temperature in the range of 70 °C to 90 °C, more preferably a temperature in the range of 70 °C to 80 °C, most preferably about 75 °C.

It was found that two pasteurisation steps enable an efficient and effective method for manufacturing milk powder.

In a further presently preferred embodiment according to the invention, the first pasteurisation and the second pasteurisation may be independently be performed for a period in the range of 5 seconds to 180 seconds, preferably for a period in the range of 10 seconds to 120 seconds, more preferably for a period in the range of 10 seconds to 100 seconds, most preferably for a period in the range of 10 seconds to 90 seconds.

In a further presently preferred embodiment according to the invention, the method further comprises the step of heating the milk substrate to a temperature in the range of 100 °C to 125 °C, preferably to a temperature in the range of 100 °C to 115 °C, more preferably to a temperature in the range of 100 °C to 105 °C, most preferably to a temperature of about 102 °C. Preferably, the step of heating the milk substrate may be performed for a period in the range of 1 second to 30 seconds, preferably a period in the range of 1 second to 20 seconds, more preferably for a period in the range of 1 second to 10 seconds, even more preferably for a period in the range of 1 second to 5 seconds, most preferably for a period of about 2 seconds.

The step of heating the milk to a temperature in the abovementioned ranges enables deactivation of the lactase used in the method according to the invention. Therefore, the hydrolysis of lactase may be stopped at the desired moment. In addition, said temperature enable to terminate active lactase which may affect the desired taste and/of texture of the milk substrate and/or milk powder.

In a further presently preferred embodiment according to the invention, the method according to the invention may be performed in a continuous manner.

Performing the method according to the invention in a continuous manner enables to increase the throughput of the method according to the invention. As a result, a more cost-effective method is achieved as the down time is reduced.

Furthermore, less germs may spread through the system used to perform the method according to the invention as a continuous process allows to have a closed system.

In a further presently preferred embodiment according to the invention, the method according to the invention further comprises the step of spray drying.

The step of spray drying enables to provide the milk powder manufactured in the method according to the invention.

An advantage of the spray drying is that the residues of the lactase may stay in the milk substrate and do not need to be removed.

In a further presently preferred embodiment according to the invention, the method according to the invention does not include membrane filtration.

An advantage of the method according to the invention is that membrane filtration is not included. As a result, the method according to the invention is more efficient as the down time due to cleaning and/or changing of the membranes is absent. Furthermore, it is known that membranes may hold germs and/or undesired particles which may contaminate the milk substrate and eventually the milk powder.

Thus, the method according to the invention without using a membrane is more efficient and effective compared to conventional methods for manufacturing milk powder comprising at most 5 dry wt.% lactose including a membrane.

In a further presently preferred embodiment according to the invention, the milk substrate may be provided with a flow speed in the range of 10 L min⁻¹ to 300 L min⁻¹, preferably with a flow speed in the range of 50 L min⁻¹ to 250 L min⁻¹, more preferably with a flow speed in the range of 100 L min⁻¹ to 200 L min⁻¹.

In a further presently preferred embodiment according to the invention, a further addition of lactase to the reactor is performed, and or the step of adding a lactase to the reactor in an amount in the range of 0.1 wt.% to 3 wt.% comprises sub steps of the addition of the lactase to the reactor.

In other words, a further addition of lactase to the reactor may be performed and/or the addition of lactase to the reactor is performed in sub-steps.

An advantage of providing a further amount of lactase to the reactor and/or providing the lactase in sub-steps is that a constant hydrolysis of the lactose is achieved.

The invention also relates to a device for manufacturing milk powder comprising at most 5 dry wt.% lactose. Said device comprises:
- a reactor comprising an inlet and an outlet, wherein the reactor is configured for holding and for heating a milk substrate to a temperature in the range of 30 °C to 80 °C;
- a lactase supply that is operatively coupled with the reactor, and that is configured for supplying a lactase amount in the range of 0.1 wt.% to 3 wt.% to the reactor; and
- an evaporator unit that is operatively coupled with the outlet of the reactor, and that is configured for heating the milk substrate to a temperature in the range of 100 °C to 125 °C for a period in the range of 1 second to 30 seconds.

The device for manufacturing milk powder comprising at most 5 dry wt.% lactose provides the same effects and advantages as those described for the method for manufacturing milk powder comprising at most 5 dry wt.% according to the invention.

In a presently preferred embodiment according to the invention, the device further comprises a control unit configured to control one or more of a temperature in the reactor, and/or a residence time, and/or an amount of lactase added to the reactor in a predeteremined period of time.

It is noted that the time refers to the residence time of the milk substrate in the reactor.

The control unit provides an improved control over the manufacturing process and, in particular, allows the various different control options to be integrated in a single control to provide the most effective manufacturing process. This will allow the manufacturing of milk powder comprising at most 5 dry wt.% lactose in a stable and efficient manner.

In a further presently preferred embodiment according to the invention, the device further comprises at least one sensor configured to measure one or more of temperature, time, concentration of lactase, concentration of lactose. It is preferred that the at least one sensor is operatively coupled to the control unit to provide measurement data to the control unit, and it is further preferred that the control unit is configured to, based on the measurement data, adjust parameters of the manufacturing process to optimize the production efficiency.

As such, the at least one sensor according to the invention may provide a feedback to an operator or computing device, such that the manufacturing of the milk powder according to the invention may be adjusted on demand. Therewith, a stable quality of the manufacturing of the milk powder is achieved. Furthermore, the combination of at least one sensor and a control unit allows a

In a further presently preferred embodiment according to the invention, the device further comprises a pasteuriser, that viewed in a process direction, is positioned upstream from the inlet of the reactor.

An advantage of pasteurizing the milk substrate before providing it to the reactor is that any impurities or contaminants have been removed before the processing of the milk substrate with the lactase. It is preferred that the pasteuriser comprises heating means to heat the milk substrate. Optionally the pasteuriser may also comprise a heat exchanger that is positioned upstream of the heating means. The milk substrate flowing to the heating means may be pre-heated in the heat exchanger using heat extracted from the pasteurised milk substrate that flows from the heating means. In other words, the heat supply side of the heat exchanger is fed by the stream of pasteurized milk substrate from the heating means. This not only reduces the energy consumption by preheating the milk substrate before the heating means, but is also serves to at least partially reduce the temperature of the pasteurized milk substrate flowing from the heating means. Therefore, an efficient and effective device for manufacturing milk powder comprising at most 5 dry wt.% lactose is achieved.

In a further presently preferred embodiment according to the invention, the heating means of the evaporator unit are one or more selected from the group of heating block, heating coil, steam injection.

In a further presently preferred embodiment according to the invention, the evaporator unit comprises a pasteuriser and/or multiple evaporators that are preferably positioned sequentially to each other.

The invention also relates to a system for manufacturing milk powder comprising at most 5 dry wt.% lactose. Said system comprises:
- a milk substrate storage tank;
- a device according to the invention that is positioned downstream of the milk substrate storage tank; and
- a spray dryer that is positioned downstream of the device.

The system for manufacturing milk powder comprising at most 5 dry wt.% lactose provides the same effects and advantages as those described for the method for manufacturing milk powder comprising at most 5 dry wt.% according to the invention, and the device for manufacturing milk powder comprising at most 5 dry wt.% lactose according to the invention. The system according to the invention provides a compact, efficient and cost-effective system to manufacture milk powder. It is noted that the term 'downstream' in respect of the device and system as mentioned in the application indicate a process direction in which the milk substrate is processed in the device and/or system.

In a presently preferred embodiment according to the invention, the system may further comprise a balance tank that, when viewed in the process direction, is positioned between the device and the spray dryer.

It is noted that the device and the balance tank may be operatively coupled, and that the balance tank and the spray dryer may be operatively coupled.

In a further presently preferred embodiment according to the invention, the system further comprises packaging means that, when viewed in a process direction, are positioned downstream of the spray dryer.

It is noted that the spray dryer and the packaging means may be operatively coupled.

The invention also relates to a milk powder obtainable by the method according to the invention comprising at most 5 wt.% lactose.

The milk powder according to the invention provides the same effects and advantages as those described for the method for manufacturing milk powder comprising at most 5 dry wt.% according to the invention, the device for manufacturing milk powder comprising at most 5 dry wt.% lactose according to the invention, and the system for manufacturing milk powder comprising at most 5 dry wt.% according to the invention.

Further advantages, features and details of the invention are elucidated on the basis of preferred embodiments thereof, wherein reference is made to the accompanying drawings, in which:
- Figure 1 shows a schematic overview of a method according to the invention;
- Figure 2 shows a schematic overview of a system according to the invention; and
- Figure 3 shows a schematic view of an example of a control unit of a system according to the invention.

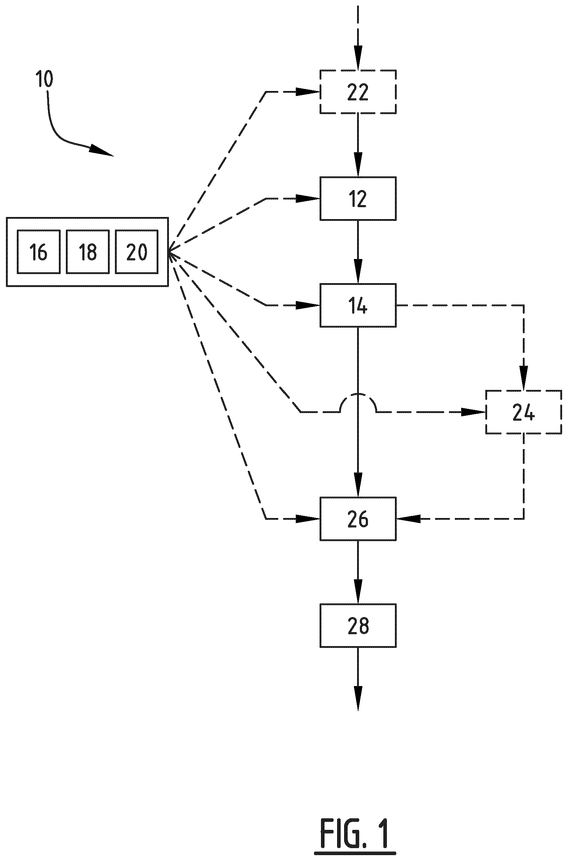

In an example, method 10 (Figure 1) for manufacturing milk powder comprising at most 5 dry wt.% lactose is provided.

In the illustrated embodiment of this example, method 10 may start with step 12 of providing a milk substrate to a reactor, wherein the milk in the reactor is heated to a temperature in the range of 30 °C to 80 °C. Said step 12 may be followed by step 14 of adding a lactase to the reactor in an amount in the range of 0.1 wt.% of 3 wt.%.

Furthermore, method 10 comprises step 16 of controlling the temperature in the reactor, and/or step 18 of controlling the addition of the lactase, and/or step 20 of controlling residence time in the reactor. Said steps may be performed independently and simultaneously with steps 12, 14, 22, 24, and 26.

Alternatively, method 10 may start with step 22 of a first pasteurisation of the milk substrate. Followed by step 12 of providing a milk substrate to a reactor, wherein the milk in the reactor is heated to a temperature in the range of 30 °C to 80 °C.

Step 14 of adding a lactase to the reactor in an amount in the range of 0.1 wt.% of 3 wt.% may be followed by step 24 of a second pasteurisation of the milk substrate and/or step 26 of heating the milk substrate to a temperature in the range of 100 °C to 125 °C, preferably to a temperature in the range of 100 °C to 115 °C, more preferably to a temperature in the range of 100 °C to 105 °C, most preferably to a temperature of about 102 °C. In addition, step 26 may be followed by step 28 of spray drying.

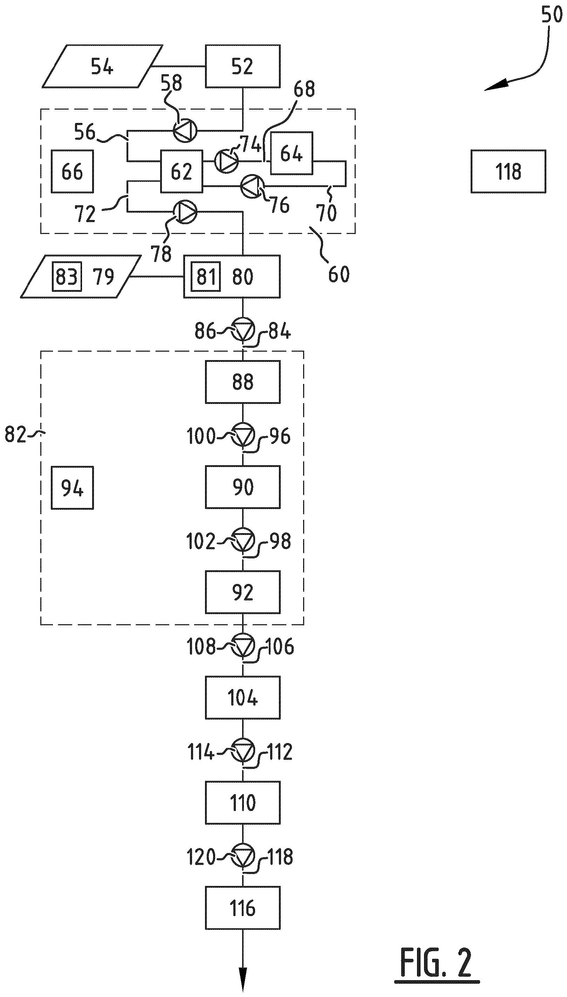

In an example of system 50 (see Figure 2) for manufacturing milk powder comprising at most 5 dry wt.% lactose, system 50 comprises the following aspects.

In the illustrated embodiment of the example of system 50, system 50 comprises milk substrate storage tank 52 which may be supplied with skimmed milk by skimmed milk supply 54. The milk in milk substrate storage tank 52 is maintained at and/or cooled to about 4 °C. Via pipe 56 and/or valve 58 the milk is provided to pasteurizer 60.

Pasteurizer 60 in this example comprises pre-heater 62, which also may be referred to as heat exchanger 62 and/or heat recovering means 62, heater 64, and sensor 66. After pasteurizer 60, the milk (also referred to as milk substrate) is provided to batch storage tank 80. Sensor 66 is configured for measuring one or more of temperature, density, flow speed, infrared. Sensor 66 may also comprise a plurality of different sensors 66, which each of the plurality being configured to measure a single parameter or multiple parameters. In the latter case, each sensor 66 is positioned at a different position in pasteurizer 60 to measure at various points in the process of pasteurisation.

Pre-heater 62, heater 64, and batch storage tank 80 are in this example connected via pipes 68, 70, 72, and/or valves 74, 76, 78 respectively. In particular, pipes 68, 70, 72 (which also may be referred to as channels or conduits) are in this example at least partially closeable by means of a respective associated valve 74, 76, 78 to regulate a flow of milk (substrate) through pasteurizer 60.

The milk substrate in batch storage tank 80 is operatively connected with lactase supply 79, wherein lactase supply 79 provides the milk substrate in batch storage tank 80 with lactase. The milk substrate is than provided from batch storage tank 80 to evaporator 82 via pipe 84 and/or valve 86. Furthermore, batch storage tank 80 comprises sensor 81. Sensor 81 is configured for measuring one or more of temperature, density, flow speed, infrared, lactose concentration. In addition, lactase supply 79 comprises sensor 83, wherein sensor 83 is configured for measuring one or more of temperature, density, flow speed, infrared, dosing of lactase. It is noted that sensors 81 and 83 may also comprise a plurality of sensors 81, 83 that are configured to measure a single one of the mentioned parameters or a multitude of the mentioned parameters. In the latter case, the each of the plurality of sensors 81, 83 is configured to measure the multitude of parametersin a different position.

Evaporator 82 comprises pasteurizer 88, direct steam injector 90, evaporator 92, and sensor 94. Pasteurizer 88, direct steam injector 90, evaporator 92 are connected via pipes 96 and 98, and/or valves 100 and 102 respectively. Sensor 94 is configured for measuring one or more of temperature, density, flow speed, infrared, lactose concentration of the various processes in the evaporator.

Evaporator 82 is operatively connected, for example via pipe 106 and/or valve 108, with balance tank 104.

In this particular example, balance tank 104 is operatively connected, for example via pipe 112 and/or valve 114, with spraydryer 110. Furthermore, spraydryer 110 is operatively connected, for example via pipe 118 and/or valve 120, with packaging means 116. Spraydryer 110 and packaging means 116 may however also be obviated.

System 50 further comprises controlling means 118 which is configured for receiving and processing input from sensors 66, 81, 83, 94. Furthermore, controlling means 118 is configured for adjusting the operation settings of the different components in system 50 based on the input of said sensors.

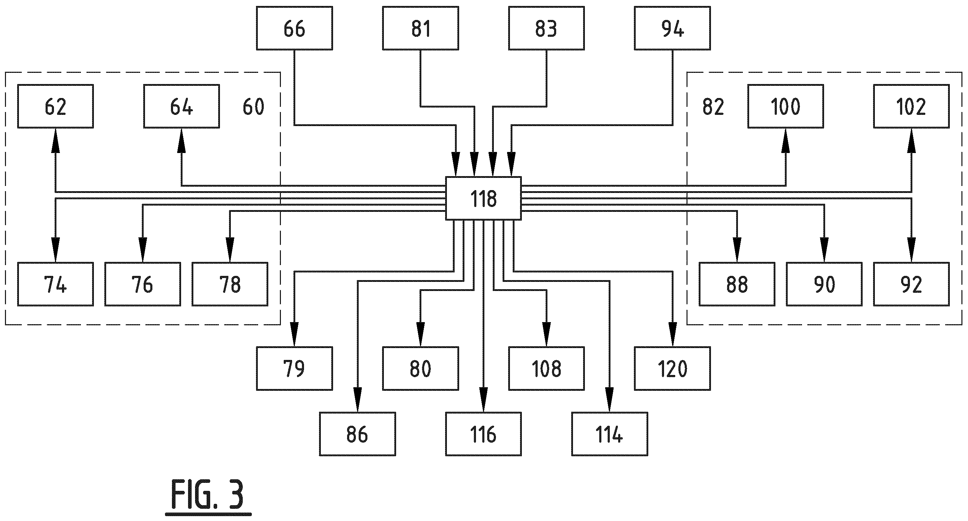

In an example of controlling means 118 or control unit 118 (see figure 3), control unit 118 is configured to control one or more different aspects of system 50. In this particular example, control unit 118 is configured to provide control signals inter alia based on sensor input from sensors 66, 81, 83, 94. Control unit 118 is connected to various hardware within system 50 to provide control signals thereto. Control unit 118 is in this example connected to for example pasteuriser 60, in particular to pre-heater 62 and heater 64, which may be controlled that is at least partially based on input from sensor 66. In addition, the flow in pasteuriser 60 is regulated using one or more of valves 58, 74, 76, 78. Control unit 118 in this example is also configured to regulate batch storage tank 80, in particular a liquid level therein, and may therefore use sensor data obtained by sensor 81. Control unit 118 in this example is also configured to regulate and/or control lactase supply 79, which is operatively connected to batch storage tank 80, to supply a predetermined amount of lactase to batch storage tank 80.

In this example, evaporator 82 is controlled by control unit 118 by means of controlling pasteurizer 88, direct steam injector 90, evaporator 92. The control may be exercised at least partially based on sensor data obtained from sensor 94. In addition, the control unit 118 may control evaporator 82 by means of regulating the flow using one or more of valves 86, 100, 102. Furthermore, in this example control unit 118 is used to control balance tank 104, for example to control the amount of (half-)product that is supplied to spraydryer 110. In this example, control unit 118 is also configured to provide control signals to packaging means 116. In particular, this may contain control signals indicating a required packaging speed to be able to package all produced product.

In an experiment, the method according to the disclosure was used to manufacture milk powder. As a result of the method, a milk powder having the characteristics as presented in table 1 was obtained. The experiment labelled 'Ref' was a reference experiment to evaluate the results of the experiments using the method according to the disclosure. It was found inter alia that the method could be used to provide milk powder having different amounts of free fat. In a first test, the amount of free at was 18%, whereas in the following test - which are provided in table 1 - the free fat percentage was reduced to between 4.2% (sample 3.2) and 6.6% (sample 1.2).

**table 1: results of analysis milk powder**

| **starttime** | **sample** | **fat %** | **protein (%)** | **ash (%)** | **moisture (%)** | **insolubility** | **Free fat %** | **WPNI (mg/kg)** |
|---|---|---|---|---|---|---|---|---|
| 10:30 | 1.2 | 42.4 | 21.3 | 4.3 | 2.3 | 0.1 | 6.6 | 2.5 |
| 12:00 | 2.2 | 42.4 | 21.1 | 4.3 | 2.2 | 0.7 | 5.4 | 2 |
| 14:00 | 3.2 | 42.6 | 20.9 | 4.3 | 2.3 | 3 | 4.2 | 2.2 |
| Ref | REF | 44 | 20 | 4.1 | 2.7 | 0.1 | 22 | 1.9 |

In addition, it was found that the method according to the disclosure provided an insolubility of the milk powder in the range of 0.1 to 3 (see table 1). It was further found that the manufactured milk powder contained less 1 dry wt.% in lactose.

The present invention is by no means limited to the above described preferred embodiments and/or experiments thereof. The rights sought are defined by the following claims within the scope of which many modifications can be envisaged.

## Claims

1. Method for manufacturing milk powder comprising at most 5 dry wt.% lactose, comprising the steps of:
- providing a milk substrate to a reactor, wherein the milk in the reactor is heated to a temperature in the range of 30 °C to 80 °C; and
- adding a lactase to the reactor in an amount in the range of 0.1 wt.% to 3 wt.%,
wherein the residence time of the milk substrate and the lactase in the reactor is in the range of 20 minutes to 180 minutes.

2. Method according to claim 1, wherein the heating of the milk in the reactor is performed at a temperature in the range of 40 °C to 70 °C, preferably at a temperature in the range of 45 °C to 60 °C, more preferably at a temperature in the range of 50 °C to 55 °C.

3. Method according to any one of the preceding claims, wherein the lactase is added in an amount in the range of 0.1 wt.% to 2 wt.%, preferably in an amount in the range of 0.2 wt.% to 1.5 wt.%, more preferably in an amount in the range of 0.3 wt.% to 1 wt.%, even more preferably in an amount in the range of 0.3 wt.% to 0.6 wt.%, and/or wherein the residence time of the milk substrate and the lactase in the reactor is in the range of 30 minutes to 150 minutes, preferably in the range of 40 minutes to 120 minutes, more preferably in the range of 50 minutes to 90 minutes, most preferably in the range of 60 minutes to 90 minutes, and/or wherein the heating of the milk substrate in the reactor is performed at a temperature in the range of 50 °C to 55 °C, wherein the step of adding lactase comprises adding lactase in an amount in the range of 0.3 wt.% to 0.6 wt.%, and wherein the residence time of the milk substrate and the lactase in the reactor is in the range of 60 minutes to 90 minutes.

4. Method according to any one of the preceding claims, wherein the lactase is a bèta-galactosidase, and/or wherein the lactase is GODO YNL-1 and/or GODO YNL-2, preferably the lactase is GODO YNL-2.

5. Method according to any one of the preceding claims, further comprising the step of controlling one or more of:
- the temperature in the reactor;
- the addition of the lactase; and/or
- controlling residence time in the reactor.

6. Method according to any one of the preceding claims, further comprising a first pasteurisation of the milk substrate and/or a second pasteurisation of the milk substrate, wherein the first pasteurisation is performed before the step of providing a milk substrate to a reactor, and the second pasteurisation is performed after the step of adding a lactase to the reactor, preferably wherein the first pasteurisation and the second pasteurisation are independently performed at a temperature in the range of 70 °C to 100 °C, preferably a temperature in the range of 70 °C to 90 °C, more preferably a temperature in the range of 70 °C to 80 °C, most preferably about 75 °C, and/or wherein the first pasteurisation and the second pasteurisation are independently performed for a period in the range of 5 seconds to 180 seconds, preferably for a period in the range of 10 seconds to 120 seconds, more preferably for a period in the range of 10 seconds to 100 seconds, most preferably for a period in the range of 10 seconds to 90 seconds.

7. Method according to any one of the preceding claims, further comprising the step of heating the milk substrate to a temperature in the range of 100 °C to 125 °C, preferably to a temperature in the range of 100 °C to 115 °C, more preferably to a temperature in the range of 100 °C to 105 °C, most preferably to a temperature of about 102 °C, preferably wherein the step of heating the milk substrate is performed for a period in the range of 1 second to 30 seconds, preferably a period in the range of 1 second to 20 seconds, more preferably for a period in the range of 1 second to 10 seconds, even more preferably for a period in the range of 1 second to 5 seconds, most preferably for a period of about 2 seconds, and/or further comprising the step of spray drying.

8. Method according to any one of the preceding claims, wherein the method is performed in a continuous manner, and/or , wherein the milk substrate is provided with a flow speed in the range of 10 L min⁻¹ to 300 L min⁻¹, preferably with a flow speed in the range of 50 L min⁻¹ to 250 L min ', more preferably with a flow speed in the range of 100 L min⁻¹ to 200 L min⁻¹.

9. Method according to any one of the preceding claims, wherein the method does not include membrane filtration.

10. Device for manufacturing milk powder comprising at most 5 dry wt.% lactose, comprising:
- a reactor comprising an inlet and an outlet, wherein the reactor is configured for holding and for heating a milk substrate to a temperature in the range of 30 °C to 80 °C;
- a lactase supply that is operatively coupled with the reactor, and that is configured for supplying a lactase amount in the range of 0.1 wt.% to 3 wt.% to the reactor; and
- an evaporator unit that is operatively coupled with the outlet of the reactor, and that is configured for heating the milk substrate to a temperature in the range of 100 °C to 125 °C for a period in the range of 1 second to 30 seconds.

11. Device according to claim 10, further comprising a control unit configured to control one or more of a temperature in the reactor, a residence time, and/or an amount of lactase added to the reactor in a predetermined period of time, and/or further comprising at least one sensor configured to measure, preferably in the reactor, one or more of a temperature, a residence time, a concentration of lactase, and/or a concentration of lactose, and/or further comprising a pasteuriser that, when viewed in a process direction, is positioned upstream from the inlet of the reactor, preferably wherein the pasteuriser comprises heating means, and optionally a heat exchanger, wherein the milk substrate is pre-heated in the heat exchanger, pasteurised in the heating means, and cooled in the heat exchanger.

12. Device according to any one of the claims 10 or 11, wherein the evaporator unit comprises heating means, wherein the heating means preferably are one or more selected from the group of heating block, heating coil, steam injection, and/or wherein the evaporator unit comprises a pasteuriser and/or multiple evaporators that are preferably positioned sequentially to each other.

13. System for manufacturing milk powder comprising at most 5 dry wt.% lactose, comprising:
- a milk substrate storage tank;
- a device according to any one of the claims 10 to 12, which is positioned downstream of the milk substrate storage tank; and
- a spray dryer that is positioned downstream of the device.

14. System according to claim 13, further comprising a balance tank that is positioned between the device and the spray dryer, and/or further comprising packaging means that are positioned downstream of the spray dryer.

15. Milk powder obtainable by the method according to any one of the claims 1 to 9 comprising at most 5 wt.% lactose.
